# EUROPEAN PATENT SPECIFICATION

(11) **EP 3 579 745 B1**
(45) Date of publication and mention of the grant of the patent: **08.03.2023**
(21) Application number: 18706963.8
(22) Date of filing: 31.01.2018
(51) Int. Cl.: A61B 5/01, A61B 5/02, A61B 5/053, A61B 5/11, A61B 5/145, A61B 5/18, A61B 5/00

(54) **ALERT SYSTEM OF THE ONSET OF A HYPOGLYCEMIA EVENT WHILE DRIVING A VEHICLE**
ALARMSYSTEM DES EINSETZENS EINES HYPOGLYKÄMIEEREIGNISSES WÄHREND DES FAHRENS EINES FAHRZEUGS
SYSTÈME D'ALERTE PRÉVENANT DE LA SURVENUE D'UN ÉVÉNEMENT HYPOGLYCÉMIQUE LORS DE LA CONDUITE D'UN VÉHICULE

(30) Priority: 10.02.2017 US 201762457389 P; 22.12.2017 US 201762609793 P
(43) Date of publication of application: 18.12.2019
(73) Proprietor: Koninklijke Philips N.V., 5656 AG Eindhoven (NL)
(72) Inventor: MENA BENITO, Maria, Estrella, 5656 AE Eindhoven (NL); BOURQUIN, Yannyk, Parulian, Julian, 5656 AE Eindhoven (NL); SHAN, Caifeng, 5656 AE Eindhoven (NL); PFUNDTNER, Stefan, 5656 AE Eindhoven (NL)
(74) Representative: Philips Intellectual Property & Standards
(86) International application number: PCT/EP2018/052325
(87) International publication number: WO 2018/145965

(56) References cited:
- US-A1- 2007 282 180
- US-A1- 2009 267 774
- US-A1- 2010 030 092
- US-A1- 2014 179 256

## Description

### FIELD OF THE INVENTION

The present invention is generally related to vehicle safety, and in particular, reducing the risk of vehicle mishaps due to hypoglycemic events.

### BACKGROUND OF THE INVENTION

Hypoglycemia (abnormally low levels of blood glucose / blood sugar) is an undesirable and potentially lethal side-effect of insulin treatment in diabetes mellitus. Hypoglycemia is frequently seen in connection with driving errors on roads and highways, including accidents with personal and/or material damage. Hypoglycemia is triggered by activation of the autonomic nervous system that gives rise to early warnings of an onset of a hypoglycemic reaction. Catecholamine release into the blood stream induces excitatory responses, including shakiness, increased heart rate, perspiration and cutaneous vasoconstriction. Neuroglycopenic symptoms affect cognitive and motor performance (e.g., difficulty concentrating, lack of coordination, visual disturbances, dizziness or light-headedness). Upon becoming aware of early autonomic indicators a diabetic individual can easily correct mild hypoglycemia.

US 2010/030092 discloses a system for estimating the onset or presence of a hypoglycemic condition in a living being according to the preamble of claims 1 and 14. US 2014/179256 discloses a vehicle system for reacting to medical conditions. US 2009/267774 discloses an automobile monitoring system for monitoring user body characteristics while the user is operating an automobile. Based on the monitored characteristics, the system may react by triggering an alarm, preventing ignition of the automobile, and/or preventing the automobile from travelling beyond a certain distance from a certain location.

U.S. Patent No. 7,052,472 discloses a system for detecting symptoms of hypoglycemia in a diabetic individual (see, e.g., Abstract), which includes a wrist strap having an electronics module (see, e.g., column 7, lines 20-27). The underside of the electronics module are electrodes that are in contact with the skin of the individual wearing the wrist strap and which are configured to provide a means for sensing perspiration as represented in the skin conductance across the electrodes (see, e.g., col. 7, lines 61-67). Bonded to one of the electrodes is a thermistor, which collectively provide a means for sensing the surface temperature of the skin at the same general location where the perspiration is sensed (see, e.g., column 8, lines 1-8). According to the Abstract, the temperature sensing system produces a temperature signal representative of a skin temperature of the diabetic individual. The conductance sensing system produces a conductance signal representative of a level of perspiration of the diabetic individual. The trending system produces a slope estimate representative of a rate of change of the skin temperature over a predetermined interval in response to the temperature signal. The threshold system produces a slope threshold representative of a hypoglycemic decline in skin temperature observed over the predetermined interval in response to the conductance signal and to the temperature signal. The alarm system produces an indication of the presence of hypoglycemic symptoms in response to the slope estimate and the slope threshold.

Though sensing skin conductivity and skin temperature are effective ways to detect the onset of a hypoglycemic event, there is a need for other and/or additional mechanisms to provide for a robust and flexible mechanism for detection of hypoglycemic events and use of that event information in situations where failure to identify the symptoms may result in harm/damage to property and health.

### SUMMARY OF THE INVENTION

One object of the present invention is to develop a hypoglycemic event detection system that can be used in a vehicle. The invention is defined by the independent claims. The dependent claims define advantageous embodiments.

In a first aspect of the invention, an apparatus is presented according to claim 1. The apparatus may be embodied as a wearable device, mobile device (e.g., smartphone), or other device(s), including a device or devices (e.g., system) located internal and/or external to the vehicle. In some embodiments, a combination of devices (e.g., a system) may implement the invention. The invention provides, among other features, a robust mechanism to avoid or mitigate the risk of vehicle mishaps due to the onset of a hypoglycemic event.

In the embodiment, the one or more input parameters comprises one or any combination of one or more physiological input parameters corresponding to the user or one or more vehicle operation related input parameters. The input parameters are sensed directly by the apparatus (e.g., using one or more embedded sensors) and/or received via a wired or wireless communications medium (e.g., from vehicle sensors, an interface, etc.). A single input parameter (e.g., a physiological parameter from glucose reading logic) may be used, or plural inputs may be used, providing a multitude of input sources to provide a comprehensive analysis of various symptoms of a hypoglycemic event that provides for a more accurate prediction.

In one embodiment, the one or more physiological input parameters comprises an indication of one or any combination of blood circulation changes, vasoconstriction changes, increase in shakiness of the user, heart rate increase, respiration increase, temperature decrease, eye motion changes, skin conductance increase, a glucose reading, or insulin intake history. For instance, a combination of these physiological parameters, or indicators of a hypoglycemic event, may be used to enable the prediction in a robust way (e.g., when some measures may be prone to error based on the measuring conditions) that inherently helps to validate the accuracy of the prediction. In one embodiment, the prediction may be based on an evaluation of input parameters (e.g., measured values) relative to a baseline health status of a user.

In the embodiment, the apparatus is configured to validate whether one or any combination of the temperature decrease or skin conductance increase is based on hypoglycemia or internal environmental conditions in the vehicle by evaluating one or more internal vehicle environmental parameters. For instance, temperature changes may be measured with a thermal camera or contact-type sensors (e.g., in contact with the skin of the user), the latter measuring the skin conductance (e.g., sweating) and/or vasoconstriction as a surrogate for skin temperature. Body temperature and sweating may be compared to temperatures, humidity, etc. in the cabin of the vehicle and/or sunlight to ensure that the effect is indeed due to hypoglycemia and not to changes in ambient/environmental conditions, again providing robustness and/or improved accuracy to the prediction.

In one embodiment, the one or more vehicle operation related input parameters comprises one or any combination of motion information about the vehicle, motion information relative to one or more other vehicles, or driving behavior of the user. For instance, a camera may be positioned proximal to and facing the driver to capture driving behavior, including erratic hand placement or movement on the steering wheel or other behavior that suggests or indicates cognitive decline due to an oncoming hypoglycemic event. In some embodiments, another camera may be placed in a frontal-looking orientation to monitor the condition and/or events on the road. By combining the frontal-looking camera information with the information from the camera monitoring the driver, the apparatus can better detect behavior that suggests cognitive decline. In some embodiments, sensors that monitor vehicle operation (e.g., steering, braking, acceleration, etc.) may be inputted to provide a similar diagnosis of symptoms.

In one embodiment, the apparatus is configured to receive the one or more input parameters while the user is driving and before the user is driving. In addition to the real-time benefit of monitoring for hypoglycemic symptoms to ensure safe operation of the vehicle, data of user behavior may be received to facilitate machine learning of baseline values for one or more of the physiological parameters and where thresholds may be set depending on deviations or deltas (changes) from the baseline values that have historically or otherwise been researched as leading to the onset of a hypoglycemic event.

In one embodiment, the apparatus is configured to trigger the device activation by communicating a signal that adjusts a device within the vehicle that effects a change in operation of the vehicle. For instance, signals communicated to actuators, motors, etc. of the vehicle may trigger added autonomous or semi-autonomous control (e.g., computer-assisted braking, lane changing, etc.) to assist the user in safe control of the vehicle to enable prompt action to alleviate the symptoms of, and stymie the onset of, a hypoglycemic event.

These and other aspects of the invention will be apparent from and elucidated with reference to the embodiment(s) described hereinafter.

### BRIEF DESCRIPTION OF THE DRAWINGS

Many aspects of the invention can be better understood with reference to the following drawings, which are diagrammatic. The components in the drawings are not necessarily to scale, emphasis instead being placed upon clearly illustrating the principles of the present invention. Moreover, in the drawings, like reference numerals designate corresponding parts throughout the several views.
FIG. 1 is a schematic diagram that illustrates an example vehicle in which a vehicle hypoglycemic event detection system is used, in accordance with an embodiment of the invention.
FIG. 2 is a schematic diagram that illustrates an example wearable device in which all or a portion of the functionality of a vehicle hypoglycemic event detection system may be implemented, in accordance with an embodiment of the invention.
FIG. 3 is a schematic diagram that illustrates an example mobile device in which all or a portion of the functionality of a vehicle hypoglycemic event detection system may be implemented, in accordance with an embodiment of the invention.
FIG. 4 is a schematic diagram that illustrates an example vehicle processing unit in which in which all or a portion of the functionality of a vehicle hypoglycemic event detection system may be implemented, in accordance with an embodiment of the invention.
FIG. 5 is a flow diagram that illustrates an example vehicle hypoglycemic event detection method, in accordance with an embodiment of the invention.

### DETAILED DESCRIPTION OF EMBODIMENTS

Disclosed herein are certain embodiments of a vehicle hypoglycemic event detection system, apparatus, and method (herein, also collectively referred to as a vehicle hypoglycemic event detection system) that detects and alerts a driver of the potential onset of a hypoglycemic event and/or activates a device that affects safe control of the vehicle at the earliest possible stages of detection, so development of hypoglycemic unawareness is avoided or mitigated and driver safety is improved. In one embodiment, an apparatus is disclosed that recognizes a user is driving and predicts a hypoglycemic event has reached a threshold probability of occurring based on one or more input parameters and alerts the user or activates a device based on the prediction. Similar functionality may achieved using a system, instructions stored on a non-transitory computer readable medium (and executed by one or more processors), or method, as disclosed hereinafter.

Digressing briefly, existing hypoglycemic event detection is not used in the context of a driving environment, and where used, has limited sensing capability and hence robustness in event detection. In contrast, certain embodiments of a vehicle hypoglycemic event detection system receives an indication that a user is driving the vehicle and also receives one or more input parameters that are used to predict that a hypoglycemic event has reached a threshold probability of occurring and trigger an alert or activate a device accordingly, enabling robust and accurate hypoglycemic event detection and facilitate safe driving. For example, detecting that a specific user is driving a vehicle allows a vehicle hypoglycemic event detection system to activate, thereby reducing power consumption associated with such a system. Additionally, individuals may have varying driving techniques and/or styles such that each individual may have a varying or learned threshold associated with various sensed parameters that may be indicative of a hypoglycemic event. Accordingly, incorporating machine learning techniques to determine a personalized threshold associated with a driver provides robust and accurate hypoglycemic event detection.

Having summarized certain features of a vehicle hypoglycemic event detection system of the present disclosure, reference will now be made in detail to the description of a vehicle hypoglycemic event detection system as illustrated in the drawings. While a vehicle hypoglycemic event detection system will be described in connection with these drawings, there is no intent to limit the vehicle hypoglycemic event detection system to the embodiment or embodiments disclosed herein. For instance, certain embodiments of a vehicle hypoglycemic event detection system may be used for users (drivers) with a diabetic condition (e.g., diabetes mellitus) or without the condition (yet may suffer from similar effects based on a hypoglycemic event while driving). Also, passengers may be monitored in some embodiments to avoid emergencies that may distract the driver, such as those lacking the capacity to become aware of the symptoms of hypoglycemia in time and causing problems for the driver. Additionally, though vehicles are described as the primary environment for certain embodiments of a vehicle hypoglycemic event detection system, other applications where safe operation is at stake may benefit from the invention, including environments involving operators handling machines (e.g., in a manufacturing setting). Further, although the description identifies or describes specifics of one or more embodiments, such specifics are not necessarily part of every embodiment, nor are all various stated advantages necessarily associated with a single embodiment or all embodiments. On the contrary, the intent is to cover all alternatives, modifications and equivalents consistent with the disclosure as defined by the appended claims. Further, it should be appreciated in the context of the present disclosure that the claims are not necessarily limited to the particular embodiments set out in the description.

Note that reference herein to detection of a hypoglycemic event, or as similarly worded, refers to the detection of one or more symptoms that are indicative of the impending onset of a hypoglycemic event.

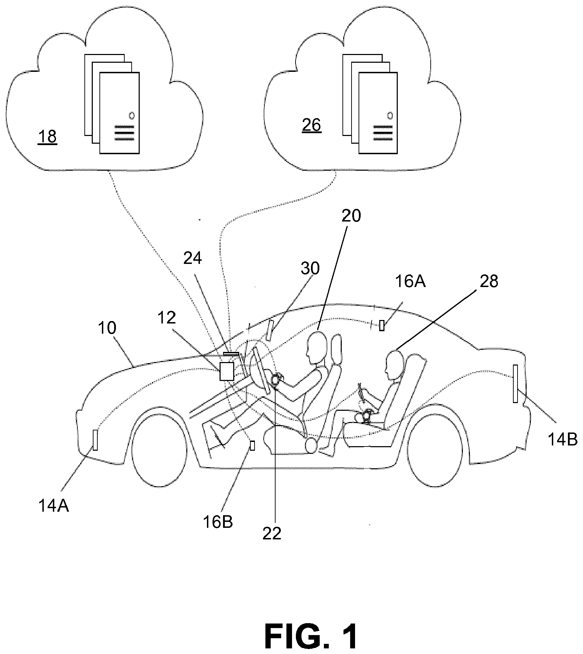

Referring now to FIG. 1, shown is an example vehicle 10 in which certain embodiments of a vehicle hypoglycemic event detection system may be implemented. It should be appreciated by one having ordinary skill in the art in the context of the present disclosure that the vehicle10 is one example among many, and that some embodiments of a vehicle hypoglycemic event detection system may be used in other types of vehicles than the type depicted in FIG. 1. FIG. 1 illustrates the vehicle 10 having a vehicle processing unit 12, external vehicle sensors 14 (e.g., front 14A and rear 14B sensors), and internal vehicle sensors 16 (e.g., 16A and 16B). Note that the quantity of sensors 14, 16 and/or vehicle processing unit 12 is illustrative of one embodiment, and that in some embodiments, fewer or greater quantities of one or more of these types of components may be used. The internal vehicle sensors 16 are located in the cabin of the vehicle 10. The external vehicle sensors 14 are located on the exterior of the vehicle 10. The external vehicle sensors 14 and internal vehicle sensors 16 are capable of communicating with the vehicle processing unit 12, such as via a wireless medium (e.g., Bluetooth, near field communications (NFC), ultrasound, and/or one of various known light-coding technologies, among others) and/or wired medium (e.g., over a controlled area network (CAN) bus or busses). The internal vehicle sensors 16 may include at least one of temperature sensors, microphones, cameras (e.g., operational in one or more different ranges of the electromagnetic spectrum), light sensors, pressure sensors, accelerometers, glucose sensors, proximity sensors, including beacons, radio frequency identification (RFID) or other coded light technologies, among other sensors. The external vehicle sensors 14 may include at least one of temperature sensors, sensors to measures precipitation and/or humidity, microphones, cameras, light sensors, pressure sensors, accelerometers, etc. In some embodiments, the vehicle 10 includes a geographic location sensor (e.g., a Global Navigation Satellite Systems (GNSS) receiver, including Global Position Systems (GPS) receiver, among others). The geographic location sensor provides location coordinates (e.g., latitude, longitude, altitude), and may assist in providing (e.g., informing of) a driving style of the user (driver).

FIG. 1 further illustrates the vehicle processing unit 12 capable of communicating with at least one cloud (e.g., cloud 1) 18. That is, the vehicle processing unit 12 is capable of communicating (e.g., via telemetry, such as according to one or more networks configured according to, say, the Global System for Mobile Communications or GSM standard, among others) with one or more devices of the cloud platform (the cloud 18). The vehicle 10 also includes vehicle sensors (e.g., whether internal or external sensors 14, 16) related to the operation of the vehicle 10 (e.g., speed, braking, turning of the steering wheel, turning of the wheels, etc.). The vehicle 10 is capable of being driven by a (human) driver 20 that primarily controls navigation (e.g., direction, vehicle speed, acceleration, etc.) of the vehicle 10, though autonomous or semi-autonomous vehicle operations may be used in certain instances.

The driver 20 may drive the vehicle 10 while wearing a wearable device 22. The wearable device 22 may include, for example, a Philips Health Watch or another fitness tracker or smartwatch. In some embodiments, the wearable device 22 may include a chest strap, arm band, ear piece, necklace, belt, clothing, headband, or another type of wearable form factor. In some embodiments, the wearable device 22 may be an implantable device, which may include biocompatible sensors that reside underneath the skin or are implanted elsewhere. The driver 20 may also wear the wearable device 22 when he is not driving the vehicle 10 (e.g., when outside the vehicle 10). The driver 20 may further drive the vehicle 10 while in possession of his or her mobile device 24 (e.g., smart phone, tablet, laptop, notebook, computer, etc.) present in the vehicle 10. The wearable device 22 is capable of communicating (e.g., via Bluetooth, 802.11, NFC, etc.) with the mobile device 24 and mobile software applications ("apps") residing thereon and/or with the vehicle processing unit 12. The mobile device 24 is capable of communicating with at least one cloud (e.g., cloud 2) 26. In some cases, the mobile device 24 is capable of communicating with the vehicle processing unit 12. At times, a passenger 28 may ride in the vehicle 10 with the driver 20, and the passenger may also possess a wearable device and/or mobile device that, in some embodiments, have functionality that is the same or similar to the wearable device 22 and/or mobile device 24 in possession of the driver 20. Further discussion of the mobile devices 24 is described below. Other examples of mobile devices may be found in International Application Publication No. WO2015084353A1, filed December 4, 2013, entitled "Presentation of physiological data," which describes an example of a user device embodied as a driver mobile device.

In general, the wearable device 22 may be in wireless communications with the vehicle processing unit 12 and with the mobile device 24. In some embodiments, the wearable device 22 may be in communication with one or both clouds 18, 26, either directly (e.g., via telemetry, such as through a cellular network) or via an intermediate device (e.g., the mobile device 24, transceiver functionality within the vehicle 10). Similarly, the vehicle processing unit 12 may be in communication with one or both clouds 18, 26. In some embodiments, all devices within the vehicle 10 may be in communication with one another and/or with the cloud(s) 18, 26.

The network enabling communications to the clouds 18, 26 may include any of a number of different digital cellular technologies suitable for use in the wireless network, including: GSM, GPRS, CDMAOne, CDMA2000, Evolution-Data Optimized (EV-DO), EDGE, Universal Mobile Telecommunications System (UMTS), Digital Enhanced Cordless Telecommunications (DECT), Digital AMPS (IS-136/TDMA), and Integrated Digital Enhanced Network (iDEN), among others. In some embodiments, communications with devices on the clouds 18, 26 may be achieved using wireless fidelity (WiFi). Access to the clouds 18, 26, which may be part of a wide area network that comprises one or a plurality of networks that in whole or in part comprise the Internet, may be further enabled through access to one or more networks including PSTN (Public Switched Telephone Networks), POTS, Integrated Services Digital Network (ISDN), Ethernet, Fiber, DSL/ADSL, WiFi, Zigbee, BT, BTLE, among others.

Clouds 18, 26 may each comprise an internal cloud, an external cloud, a private cloud, or a public cloud (e.g., commercial cloud). For instance, a private cloud may be implemented using a variety of cloud systems including, for example, Eucalyptus Systems, VMWare vSphere^{®}, or Microsoft^{®} HyperV. A public cloud may include, for example, Amazon EC2^{®}, Amazon Web Services^{®}, Terremark^{®}, Savvis^{®}, or GoGrid^{®}. Cloud-computing resources provided by these clouds may include, for example, storage resources (e.g., Storage Area Network (SAN), Network File System (NFS), and Amazon S3^{®}), network resources (e.g., firewall, load-balancer, and proxy server), internal private resources, external private resources, secure public resources, infrastructure-as-a-services (laaSs), platform-as-a-services (PaaSs), or software-as-a-services (SaaSs). The cloud architecture may be embodied according to one of a plurality of different configurations. For instance, if configured according to MICROSOFT AZURE^{™}, roles are provided, which are discrete scalable components built with managed code. Worker roles are for generalized development, and may perform background processing for a web role. Web roles provide a web server and listen for and respond to web requests via an HTTP (hypertext transfer protocol) or HTTPS (HTTP secure) endpoint. VM roles are instantiated according to tenant defined configurations (e.g., resources, guest operating system). Operating system and VM updates are managed by the cloud. A web role and a worker role run in a VM role, which is a virtual machine under the control of the tenant. Storage and SQL services are available to be used by the roles. As with other clouds, the hardware and software environment or platform, including scaling, load balancing, etc., are handled by the cloud.

In some embodiments, services of the clouds 18, 26 may be implemented in some embodiments according to multiple, logically-grouped servers (run on server devices), referred to as a server farm. The devices of the server farm may be geographically dispersed, administered as a single entity, or distributed among a plurality of server farms, executing one or more applications on behalf of or in conjunction with one or more of the wearable device 22, the mobile device 24, and/or the vehicle processing unit 12. The devices within each server farm may be heterogeneous. One or more of the devices of the server farm may operate according to one type of operating system platform (e.g., WINDOWS NT, manufactured by Microsoft Corp. of Redmond, Wash.), while one or more of the other devices may operate according to another type of operating system platform (e.g., Unix or Linux). The group of devices of the server farm may be logically grouped as a farm that may be interconnected using a wide-area network (WAN) connection or medium-area network (MAN) connection, and each device may each be referred to as (and operate according to) a file server device, application server device, web server device, proxy server device, or gateway server device.

In some embodiments, the vehicle 10 also includes at least one camera 30. The camera 30 is capable of communicating with at least one of the vehicle processing unit 12, the wearable device 22, the mobile device 24, and/or the cloud (e.g., cloud 18 and/or cloud 26). Though depicted as secured to an interior structure of the vehicle 10, as is described below, camera functionality may be implemented in the wearable device 22 and/or the mobile device 24, in addition to, or in lieu of, the camera 30. Further, in some embodiments, multiple cameras 30 of the same or different functionality may be used. The camera 30 may be located/positioned to view the driver's face and torso. For instance, the camera(s) 30 may monitor a driver's driving behavior or style (e.g., speed, acceleration, breaking, cornering, odd movements, and distance to other cars) as an estimation of motor and/or cognitive performance. The driving style (a vehicle operation related parameter) can also be measured with GNSS (e.g., GPS) functionality found in the internal sensors 16, the wearable device 22, and/or the mobile device 24 with or without the use of other sensors, for instance, accelerometers. In some embodiments, the camera 30 includes a thermal imaging camera (e.g., to measure the skin temperature of the driver 20). In some embodiments, the camera 30 comprises a vital signs camera, such as the Philips Vital Signs Camera. The Vital Signs Camera 30 remotely measures heart and breathing rate using a standard, infrared (IR) based camera by sensing changes in skin color and body movement (e.g., chest movement). For instance, whenever the heart beats, the skin color changes because of the extra blood running through the vessels. Algorithms residing within the Vital Signs Camera 30 detect these tiny skin color changes, amplify the signals, and calculate a pulse rate signal by analyzing the frequency of the skin color changes. For respiration, the Vital Signs Camera 30 focuses on the rise and fall of the chest and/or abdomen, amplifying the signals using algorithms and determining an accurate breathing rate. Note that in some cases, the respiration rate may be determined from signals indicating skin color changes. The Vital Signs Camera 30 is also motion robust, using facial tracking to obtain an accurate reading during motion. More particularly in relation to detection of hypoglycemic events, the Vital Signs Camera 30 is configured to measure one or any combination of a driver's physiological signs or symptoms related to hypoglycemic events, including blood circulation (vasoconstriction), heart rate (palpitation) and respiration rate (faster and irregular). Other visual signs of the driver related to detection of a hypoglycemic event that may be monitored by the Vital Signs Camera 30 include changes in the skin (e.g., perspiration, clammy skin, and/or pale skin), eye motion (motor and cognitive performance), and/or physiological tremors and/or shakiness of the driver 20.

The wearable device 22 includes one or more of an accelerometer, photoplethysmograpm (PPG) sensor, sensors for detecting electrodermal activity (EDA) (e.g., detects a variation in the electrical characteristics of the skin, including skin conductance, galvanic skin response, electrodermal response), blood pressure cuff, blood glucose monitoring, electrocardiogram sensor, step counter sensor, gyroscope, SpO2 sensor (e.g., providing an estimate of arterial oxygen saturation), respiration sensor, posture sensor, stress sensor, galvanic skin response sensor, temperature sensor, pressure sensor, light sensor, and/or other physiological parameter sensors. The sensors of the wearable device 22 may include functionality of the camera 30 for detecting various physiological parameters pertaining to a hypoglycemic event, as described above, including blood circulation, heart rate, respiration rate, changes in the skin (e.g., skin conductance, including from sweating), and use vasoconstriction as a surrogate for skin temperature. In some embodiments, camera functionality in the wearable device 22 may include a thermal imaging camera for skin temperature measurements. In some embodiments, the wearable device 22 comprises one or more accelerometers for use in the detection of hypoglycemic events. For instance, the accelerometer of wearable device 22 may be used to detect (and measure) a physiological tremor signal. In other words the wearable device 22 may recognize a tremor and/or shakiness of the driver 20 from a hypoglycemic event (as opposed to from vehicle motion, which may be excluded through algorithms running in the wearable device 22). The wearable device 22 is capable of sensing signals related to heart rate, heart rate variability, respiration rate, pulse transit time, blood pressure, temperature (including functionality for excluding environmental temperature), among other physiological parameters. Other possible parameters and sensors are described in Table 1 of US Patent No. 8390546, filed September 13, 2004, and entitled "System for monitoring and managing body weight and other physiological conditions including iterative and personalized planning, intervention and reporting capability."

The mobile device 24 may include functionality of the camera 30 and/or one or more other sensing functionality, including GNSS functionality.

In some embodiments, the sensors and/or sensor functionality described above for the wearable device 22 (of which one or more may reside in the mobile device 24 in some embodiments) may be integrated in structures of the vehicle 10 as internal sensors 16 (including the camera 30). Likewise, one or more of the aforementioned functionality of the internal sensors 16 (including the camera 30) may be included in the wearable device 22 and/or the mobile device 24. In one embodiment, a series of sensing systems and touch and grip sensors can be integrated in the steering wheel of the vehicle 10 to detect changes in physiological signals of driver (e.g., heart rate acceleration, perspiration, clammy skin, etc.). In some embodiments, touch or force handles can be integrated in the steering wheel to detect tremors and/or shakiness. In some embodiments, grip sensors can be integrated into the shifting gears to detect changes in physiological signals and hand tremors of the driver 20. In some embodiments, motion sensors (e.g. accelerometers) and sensors in the seat may be used to monitor shaking and/or perspiration of the driver 20. In some embodiments, a glucose reading device can be connected to the steering wheel to get a much better estimation of the risk of hypoglycemia in a diabetic driver. In some embodiments, the time of an insulin intake event by the driver 20 is recorded from an electronic insulin dispenser or manually in an app interface of the mobile device 24 and/or in the wearable device 22. This data may be used in the estimation of onset of a potential hypoglycemia event.

Note that in some embodiments, measurements from the sensors from the wearable device 22, mobile device 24, internal sensors 16, external sensors 14, and/or camera 30 may be fused together (e.g., used in combination as a basis for the prediction of a hypoglycemic event). For instance, the measurements may be collected by the vehicle processing unit 12, the wearable device 22, the mobile device 24, one or more devices of the cloud(s) 18, 26, and used to make a prediction about the probability that a hypoglycemic event is about to occur.

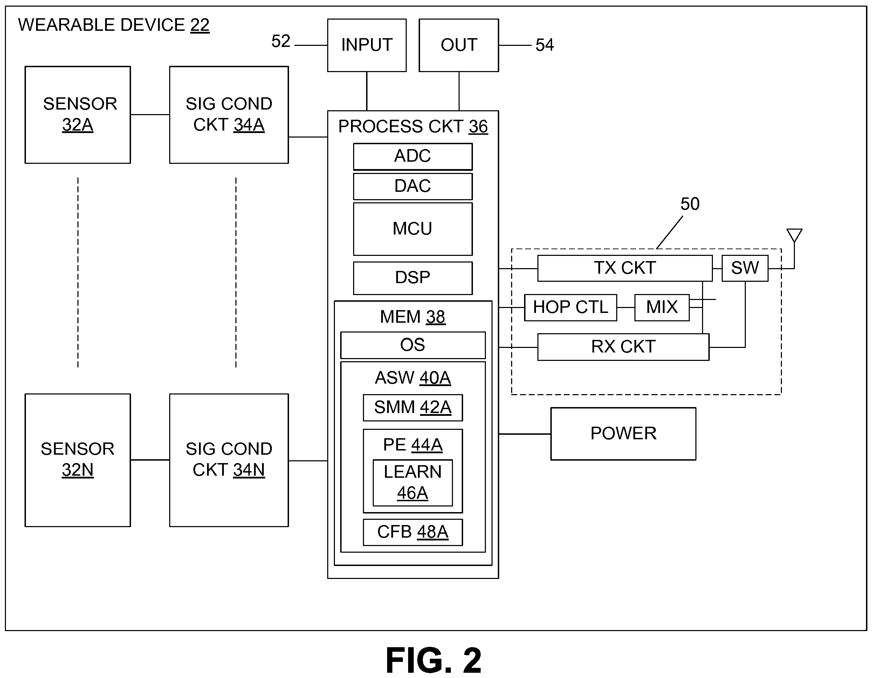

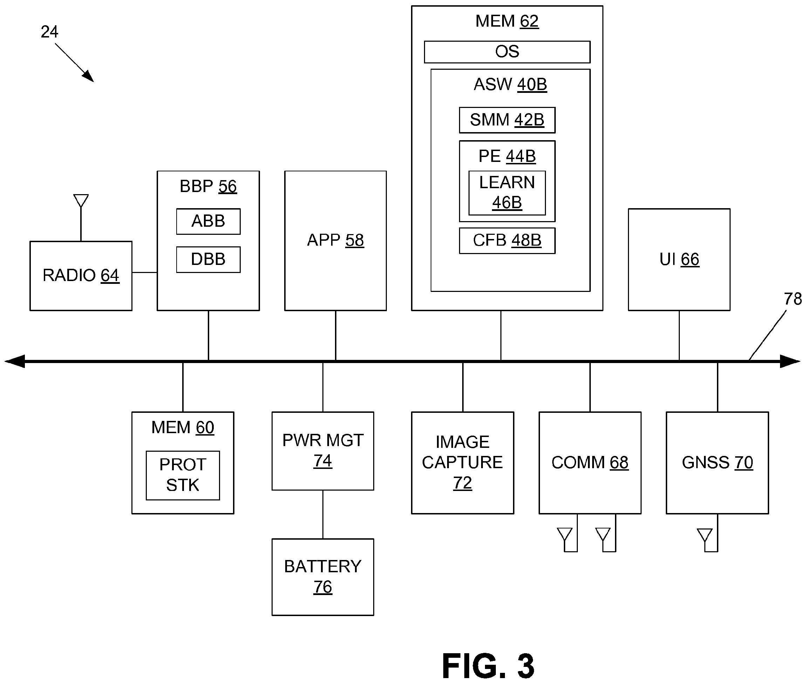

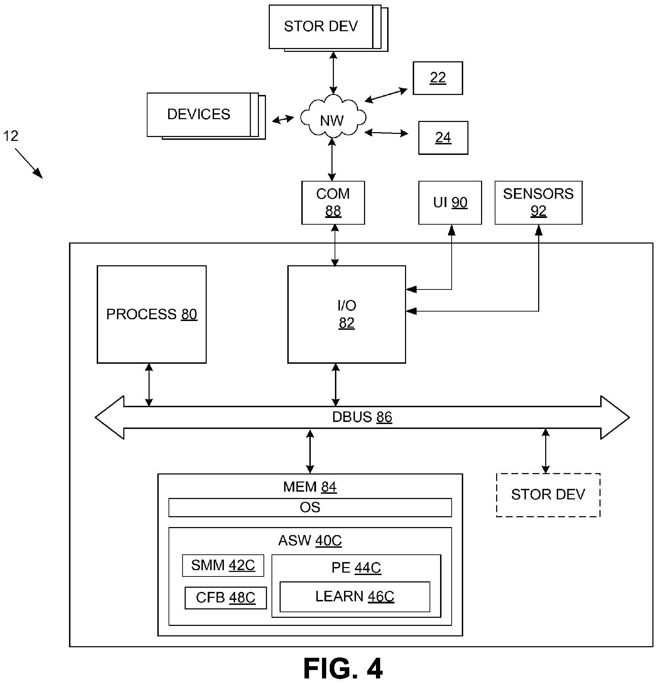

As indicated above, processing for certain embodiments of the vehicle hypoglycemic event detection system may be performed in one or any combination of the vehicle processing unit 12, a cloud(s) (e.g., one or more devices of the clouds 18 and/or 26), the wearable device 22, or the mobile device 24. Various embodiments of the invention propose to overcome the lack of hypoglycemic event detection while operating a vehicle, and doing so in a robust and accurate way to avoid or mitigate false alarms and/or to ensure reliability in detection. In the description that follows, primary processing functionality for certain embodiments of a vehicle hypoglycemic event detection system is described as being achieved in the wearable device 22 (FIG. 2), mobile device 24 (FIG. 3), and then the vehicle processing unit 12 (FIG. 4), with the understanding that some or all of the processing may be offloaded to a device or devices (e.g., of a similar architecture to that described for the vehicle processing unit 12) of the cloud(s) 18, 26 in some embodiments, or implemented using a combination of the above devices.

Attention is now directed to FIG. 2, which illustrates an example wearable device 22 in which all or a portion of the functionality of a vehicle hypoglycemic event detection system may be implemented. In particular, FIG. 2 illustrates an example architecture (e.g., hardware and software) for the wearable device 22. It should be appreciated by one having ordinary skill in the art in the context of the present disclosure that the architecture of the wearable device 22 depicted in FIG. 2 is but one example, and that in some embodiments, additional, fewer, and/or different components may be used to achieve similar and/or additional functionality. In one embodiment, the wearable device 22 comprises a plurality of sensors 32 (e.g., 32A-32N), one or more signal conditioning circuits 34 (e.g., SIG COND CKT 34A - SIG COND CKT 34N) coupled respectively to the sensors 32, and a processing circuit 36 (comprising one or more processors) that receives the conditioned signals from the signal conditioning circuits 34. In one embodiment, the processing circuit 36 comprises an analog-to-digital converter (ADC), a digital-to-analog converter (DAC), a microcontroller unit (MCU), a digital signal processor (DSP), and memory (MEM) 38. In some embodiments, the processing circuit 36 may comprise fewer or additional components than those depicted in FIG. 2. For instance, in one embodiment, the processing circuit 36 may consist entirely of the microcontroller unit. In some embodiments, the processing circuit 36 may include the signal conditioning circuits 34.

The memory 38 comprises an operating system (OS) and application software (ASW) 40A, which in one embodiment comprises one or more functionality of a vehicle hypoglycemic event detection system. In some embodiments, additional software may be included for enabling physical and/or behavioral tracking, among other functions. In the depicted embodiment, the application software 40A comprises a sensor measurement module (SMM) 42A for processing signals received from the sensors 32, a prediction engine (PE) 44A for predicting a probability of a hypoglycemic event occurring, a learning (LEARN) module 46A for learning baseline values and/or thresholds for the hypoglycemic factors, and a communications/feedback (CFB) module (FM) 48A for activating or triggering circuitry of the wearable device 22 and/or other devices to alert the user of the risk of a hypoglycemic event and/or to actuate one or more devices that are used to effect a change in operation of the vehicle 10 (FIG. 1). In some embodiments, additional modules used to achieve the disclosed functionality of a vehicle hypoglycemic event detection system, among other functionality, may be included, or one or more of the modules 42A-48A may be separate from the application software 40A or packaged in a different arrangement than shown relative to each other. In some embodiments, fewer than all of the modules 42A-48A may be used in the wearable device 22.

As used herein, the term "module" may be understood to refer to computer executable software, firmware, hardware, and/or various combinations thereof. It is noted there where a module is a software and/or firmware module, the module is configured to affect the hardware elements of an associated system. It is further noted that the modules shown and described herein are intended as examples. The modules may be combined, integrated, separated, or duplicated to support various applications. Also, a function described herein as being performed at a particular module may be performed at one or more other modules and by one or more other devices instead of or in addition to the function performed at the particular module. Further, the modules may be implemented across multiple devices or other components local or remote to one another. Additionally, the modules may be moved from one device and added to another device, or may be included in both devices.

The sensor measurement module 42A comprises executable code (instructions) to process the signals (and associated data) measured by the sensors 32. For instance, the sensors 32 may measure one or more input parameters corresponding to physiological measurements of the driver from the sensors 32 (and/or other input). As indicated above, these measurements, also referred to as indicators (of a hypoglycemic event), may include blood circulation, heart rate, respiration rate, skin conductance, skin temperature, eye motion, tremors/shakiness, torso or facial movements, glucose measurements, among other data.

The prediction engine 44A comprises executable code (instructions) to predict whether a hypoglycemic event has reached a threshold probability of occurring (e.g., when reaching 95% probability, an alert is triggered). In some embodiments, multiple threshold probabilities may be used to provide a progressive warning to the user (e.g., lower to higher risks). The prediction engine 44A receives the indicators from the sensor measurement module 42A and/or from other input sources (e.g., received over a wireless connection, inputted manually to the wearable device 22, etc.) and, in one embodiment, implements a rules-based approach to determine whether one or more of the indicators have deviated a respective threshold amount from their respective baseline values. In one embodiment, the prediction engine 44A comprises a learning (LEARN) module 46A that is configured to learn baseline values for the indicators. In one embodiment, default values (e.g., based on population statistics, public medical/research data, etc.) may initially be used as baseline values, and the learning module 46A adjusts these values over time as measurements are collected for the user to enable baseline values for the indicators that are tailored/personalized to the user. In some embodiments, the initial baseline values may be based on user-specific values acquired (with appropriate permissions) from medical data structures containing the medical history of the user and/or as collected in everyday use, and in some embodiments, the baseline values may be based on a combination of default values, personalized values (both initially and as learned), and historical data collected over time for the user. Deviations or deltas from the baseline values may indicate a higher probability that a hypoglycemic event is about to occur or has occurred. For instance, vasoconstriction, heart palpitations, faster and/or irregular respiration rate, perspiration, clammy skin, pale skin, tremors and/or shakiness, etc. may be manifestations of these deviations. In one embodiment, the prediction engine 44A uses thresholds for each of the indicators to determine whether the deviations or deltas from the respective baseline values have reached a level indicating a high probability of a hypoglycemic event (or, in some embodiments, multiple threshold probabilities may be used to progressively alert the user of the trending risk). The thresholds may be learned (e.g., via learning module 46A), including from past instances of the user experiencing a hypoglycemic event and/or as determined from public data structures (e.g., research, medical) and/or private, historical medical data of the user (and/or family members, including history of hypoglycemia, diabetes, etc.). The thresholds may be learned using machine learning techniques such that each user has user-specific threshold values associated with the user's driving style and techniques. For example, machine learning techniques such as decision tree learning, neural networks, deep learning, support vector machines (SVMs), Bayesian networks, and the like may be used to classify and/or identify a particular threshold for driver/automobile behaviors (e.g., driver physiological data, driver movement data, and automobile data) to identify whether these behaviors are indicative of a hypoglycemic event or predictive of a hypoglycemic event.

The prediction engine 44A predicts that a hypoglycemic event is about to occur (e.g., has reached a threshold probability of occurring). The threshold probability may be a dynamic value that is determined based on a composite (e.g., sum) of the deviation or delta thresholds for all detected indicators. For instance, the prediction engine 44A may use a rules-based approach, where depending on which indicators have been detected the respective threshold deviation values are summed to determine the threshold probability. In some instances, a single indicator may be indicative of a risk of a hypoglycemic event that is about to occur, and hence the probability threshold is equal to the deviation threshold for that indicator. In some embodiments, the probability threshold may be based on a weighted summation of the deviation thresholds. In some embodiments, as described above, multiple probability thresholds (with different intensity of alerts and/or actions) may be used. In some embodiments, other mechanisms may be used to determine a probability threshold.

The communications/feedback module 48A comprises executable code (instructions) that receives an indication from the prediction engine 44A of the meeting or exceeding of the threshold probability, and triggers an alert and/or activation of a device affecting vehicle operations. The alerts serve to provide real-time (e.g., immediate) feedback that the user is about to experience a hypoglycemic event, achieving hypoglycemia awareness and enabling the user to take remedial actions. In one embodiment, the communications/feedback module 48A may activate an output interface (described below) of the wearable device 22, which in turn may result in visual, audible, and/or haptic feedback (e.g., alerts) to the user. In some embodiments, the communications/feedback module 48A may, in conjunction with wireless communications circuitry (described below), communicate (e.g., wirelessly) a signal to another device, which in turn provides for visual, audible, and/or haptic feedback to the user (driver). For instance, the device receiving the wireless signal may include the vehicle processing unit 12 (FIG. 1, such as for presentation on the vehicle dashboard or alert through vehicle speakers), the mobile device 24 (FIG. 1, such as for vibration motor activation and/or visual feedback on the display screen of the mobile device 24), a heads-up display or Google glasses worn by the user, etc. In some embodiments, the communications/feedback module 48A may, in conjunction with communications circuitry, send a command signal to the vehicle processing unit 12, which in turn activates one or more vehicle components/devices that involve control of the vehicle or vehicle operations. For instance, the vehicle components may involve actuators or motors that cause braking (increasing the distance between a vehicle ahead), or activates autonomous or semi-autonomous features that determine whether the lane adjacent the vehicle is clear and activate turning signals and steering wheel signals and actuators for brakes to move the vehicle over to the side of the road. Other actions may be taken to ensure safe operation of the vehicle and/or to facilitate preventing the user from entering into a condition of hypoglycemic unawareness (e.g., where the hypoglycemic event has now actually occurred). The communications/feedback module 48A comprises executable code (instructions) to enable a communications circuit 50 of the wearable device 22 to operate according to one or more of a plurality of different communication technologies (e.g., NFC, Bluetooth, Zigbee, 802.11, Wireless-Fidelity, GSM, etc.) to receive from, and/or transmit data to, one or more devices (e.g., other wearable devices, mobile devices, cloud devices, vehicle processing unit, cameras, actuators, motors, etc.) internal to the vehicle 10 or external to the vehicle 10.

As indicated above, in one embodiment, the processing circuit 36 is coupled to the communications circuit 50. The communications circuit 50 serves to enable wireless communications between the wearable device 22 and other devices within or external to the vehicle 10 (FIG. 1). The communications circuit 50 is depicted as a Bluetooth (BT) circuit, though not limited to this transceiver configuration. For instance, in some embodiments, the communications circuit 50 may be embodied as any one or a combination of an NFC circuit, Wi-Fi circuit, transceiver circuitry based on Zigbee, BT low energy, 802.11, GSM, LTE, CDMA, WCDMA, among others such as optical or ultrasonic based technologies. In some embodiments, plural transceiver circuits according to more than one of the communication specifications/standards described above may be used.

The processing circuit 36 is further coupled to input/output (I/O) devices or peripherals, including an input interface 52 (INPUT) and an output interface 54 (OUT). In some embodiments, an input interface 52 and/or an output interface 54 may be omitted, or functionality of both may be combined into a single component. The input and output interfaces 52, 54 are described further below.

Note that in some embodiments, functionality for one or more of the aforementioned circuits and/or software may be combined into fewer components/modules, or in some embodiments, further distributed among additional components/modules or devices. For instance, the processing circuit 36 may be packaged as an integrated circuit that includes the microcontroller (microcontroller unit or MCU), the DSP, and memory 38, whereas the ADC and DAC may be packaged as a separate integrated circuit coupled to the processing circuit 36. In some embodiments, one or more of the functionality for the above-listed components may be combined, such as functionality of the DSP performed by the microcontroller.

As indicated above, the sensors 32 comprise one or any combination of sensors capable of measuring physiological, motor and/or cognitive, and external (e.g., environmental) parameters. For instance, typical physiological parameters include heart rate, heart rate variability, heart rate recovery, blood flow rate, blood circulation, activity level, muscle activity (including tremors and/or shakes), muscle tension, blood volume, blood pressure, blood oxygen saturation, respiratory rate, perspiration, skin temperature, electrodermal activity (skin conductance response, galvanic skin response, electrodermal response, etc.), body weight, and body composition (e.g., body mass index or BMI), articulator movements (especially during speech), and eye movement (for cognitive and/or motor sensing). The sensors 32 may also include global navigation satellite system (GNSS) sensors/receiver (e.g., to monitor the driving style of the user). The sensors 32 may also include inertial sensors (e.g., gyroscopes) and/or magnetometers, which may assist in the determination of driving behavior. In some embodiments, GNSS sensors (e.g., GNSS receiver and antenna(s)) may be included in the mobile device 24 (FIG. 1) and/or the vehicle 10 (FIG. 1), in addition to, or in lieu of, those residing in the wearable device 22. In some embodiments, GNSS functionality may be achieved via the communications circuit 50 or other circuits coupled to the processing circuit 36. The sensors 32 may also include flex and/or force sensors (e.g., using variable resistance), electromyographic sensors, electrocardiographic sensors (e.g., EKG, ECG), magnetic sensors, photoplethysmographic (PPG) sensors, bio-impedance sensors, infrared proximity sensors, acoustic/ultrasonic/audio sensors, a strain gauge, galvanic skin/sweat sensors, pH sensors, temperature sensors, cameras (e.g., thermal and/or with Vital Sign functionality as described above), and photocells. The sensors 32 may include other and/or additional types of sensors for the detection of environmental parameters and/or conditions, for instance, barometric pressure, humidity, outdoor temperature, pollution, noise level, etc. Note that one or more of the sensors 32 may be constructed based on piezoelectric, piezoresistive or capacitive technology in a microelectromechanical system (MEMS) infrastructure.

The signal conditioning circuits 34 include amplifiers and filters, among other signal conditioning components, to condition the sensed signals including data corresponding to the sensed physiological parameters and/or location signals before further processing is implemented at the processing circuit 36. Though depicted in FIG. 2 as respectively associated with each sensor 32, in some embodiments, fewer signal conditioning circuits 34 may be used (e.g., shared for more than one sensor 32). In some embodiments, the signal conditioning circuits 34 (or functionality thereof) may be incorporated elsewhere, such as in the circuitry of the respective sensors 32 or in the processing circuit 36 (or in components residing therein). Further, although described above as involving unidirectional signal flow (e.g., from the sensor 32 to the signal conditioning circuit 34), in some embodiments, signal flow may be bi-directional. For instance, in the case of optical measurements, the microcontroller may cause an optical signal to be emitted from a light source (e.g., light emitting diode(s) or LED(s)) in or coupled to the circuitry of the sensor 32, with the sensor 32 (e.g., photocell) receiving the reflected/refracted signals.

The communications circuit 50 is managed and controlled by the processing circuit 36 (e.g., executing the communications/feedback module 48A). The communications circuit 50 is used to wirelessly interface with one or more devices within and/or external to the vehicle 10 (FIG. 1). In one embodiment, the communications circuit 50 may be configured as a Bluetooth transceiver, though in some embodiments, other and/or additional technologies may be used, such as Wi-Fi, GSM, LTE, CDMA and its derivatives, Zigbee, NFC, among others. In the embodiment depicted in FIG. 2, the communications circuit 50 comprises a transmitter circuit (TX CKT), a switch (SW), an antenna, a receiver circuit (RX CKT), a mixing circuit (MIX), and a frequency hopping controller (HOP CTL). The transmitter circuit and the receiver circuit comprise components suitable for providing respective transmission and reception of an RF signal, including a modulator/demodulator, filters, and amplifiers. In some embodiments, demodulation/modulation and/or filtering may be performed in part or in whole by the DSP. The switch switches between receiving and transmitting modes. The mixing circuit may be embodied as a frequency synthesizer and frequency mixers, as controlled by the processing circuit 36. The frequency hopping controller controls the hopping frequency of a transmitted signal based on feedback from a modulator of the transmitter circuit. In some embodiments, functionality for the frequency hopping controller may be implemented by the microcontroller or DSP. Control for the communications circuit 50 may be implemented by the microcontroller, the DSP, or a combination of both. In some embodiments, the communications circuit 50 may have its own dedicated controller that is supervised and/or managed by the microcontroller.

In one example operation for the communications circuit 50, a signal (e.g., at 2.4 GHz) may be received at the antenna and directed by the switch to the receiver circuit. The receiver circuit, in cooperation with the mixing circuit, converts the received signal into an intermediate frequency (IF) signal under frequency hopping control attributed by the frequency hopping controller and then to baseband for further processing by the ADC. On the transmitting side, the baseband signal (e.g., from the DAC of the processing circuit 36) is converted to an IF signal and then RF by the transmitter circuit operating in cooperation with the mixing circuit, with the RF signal passed through the switch and emitted from the antenna under frequency hopping control provided by the frequency hopping controller. The modulator and demodulator of the transmitter and receiver circuits may perform frequency shift keying (FSK) type modulation/demodulation, though not limited to this type of modulation/demodulation, which enables the conversion between IF and baseband. In some embodiments, demodulation/modulation and/or filtering may be performed in part or in whole by the DSP. The memory 38 stores the communications/feedback module 48A, which when executed by the microcontroller, controls the Bluetooth (and/or other protocols) transmission/reception.

Though the communications circuit 50 is depicted as an IF-type transceiver, in some embodiments, a direct conversion architecture may be implemented. As noted above, the communications circuit 50 may be embodied according to other and/or additional transceiver technologies.

The processing circuit 36 is depicted in FIG. 2 as including the ADC and DAC. For sensing functionality, the ADC converts the conditioned signal from the signal conditioning circuit 34 and digitizes the signal for further processing by the microcontroller and/or DSP. The ADC may also be used to convert analogs inputs that are received via the input interface 52 to a digital format for further processing by the microcontroller. The ADC may also be used in baseband processing of signals received via the communications circuit 50. The DAC converts digital information to analog information. Its role for sensing functionality may be to control the emission of signals, such as optical signals or acoustic signals, from the sensors 32. The DAC may further be used to cause the output of analog signals from the output interface 54. Also, the DAC may be used to convert the digital information and/or instructions from the microcontroller and/or DSP to analog signals that are fed to the transmitter circuit. In some embodiments, additional conversion circuits may be used.

The microcontroller and the DSP provide processing functionality for the wearable device 22. In some embodiments, functionality of both processors may be combined into a single processor, or further distributed among additional processors. The DSP provides for specialized digital signal processing, and enables an offloading of processing load from the microcontroller. The DSP may be embodied in specialized integrated circuit(s) or as field programmable gate arrays (FPGAs). In one embodiment, the DSP comprises a pipelined architecture, which comprises a central processing unit (CPU), plural circular buffers and separate program and data memories according to a Harvard architecture. The DSP further comprises dual busses, enabling concurrent instruction and data fetches. The DSP may also comprise an instruction cache and I/O controller, such as those found in Analog Devices SHARC^{®} DSPs, though other manufacturers of DSPs may be used (e.g., Freescale multi-core MSC81xx family, Texas Instruments C6000 series, etc.). The DSP is generally utilized for math manipulations using registers and math components that may include a multiplier, arithmetic logic unit (ALU, which performs addition, subtraction, absolute value, logical operations, conversion between fixed and floating point units, etc.), and a barrel shifter. The ability of the DSP to implement fast multiply-accumulates (MACs) enables efficient execution of Fast Fourier Transforms (FFTs) and Finite Impulse Response (FIR) filtering. Some or all of the DSP functions may be performed by the microcontroller. The DSP generally serves an encoding and decoding function in the wearable device 22. For instance, encoding functionality may involve encoding commands or data corresponding to transfer of information. Also, decoding functionality may involve decoding the information received from the sensors 32 (e.g., after processing by the ADC).

The microcontroller comprises a hardware device for executing software/firmware, particularly that stored in memory 38. The microcontroller can be any custom made or commercially available processor, a central processing unit (CPU), a semiconductor based microprocessor (in the form of a microchip or chip set), a macroprocessor, or generally any device for executing software instructions. Examples of suitable commercially available microprocessors include Intel's^{®} Itanium^{®} and Atom^{®} microprocessors, to name a few non-limiting examples. The microcontroller provides for management and control of the wearable device 22.

The memory 38 (also referred to herein as a non-transitory computer readable medium) can include any one or a combination of volatile memory elements (e.g., random access memory (RAM, such as DRAM, SRAM, SDRAM, etc.)) and nonvolatile memory elements (e.g., ROM, Flash, solid state, EPROM, EEPROM, etc.). Moreover, the memory 38 may incorporate electronic, magnetic, and/or other types of storage media. The memory 38 may be used to store sensor data over a given time duration and/or based on a given storage quantity constraint for later processing. For instance, the memory 38 may comprise a data structure of indicators of hypoglycemia and respective threshold values, historical data collected via the sensors 32 and/or via other mechanisms (e.g., manual data entry, wireless signal, etc.). In some embodiments, such data may be stored elsewhere (e.g., in the cloud(s) 18, 26, FIG. 1) and accessed as needed.

The software in memory 38 may include one or more separate programs, each of which comprises an ordered listing of executable instructions for implementing logical functions. In the example of FIG. 2, the software in the memory 38 includes a suitable operating system and the application software 40A, which in one embodiment, comprises the sensor measurement module 42A, prediction engine 44A (including the learning module 46A), and the communications/feedback module 48A, as described above.

The operating system essentially controls the execution of computer programs, such as the application software 40A and associated modules 42A-48A, and provides scheduling, input-output control, file and data management, memory management, and communication control and related services. The memory 38 may also include user data, including weight, height, age, gender, goals, body mass index (BMI) that may be used by the microcontroller executing executable code to accurately interpret the measured parameters. The user data may also include historical data relating past recorded data to prior contexts, including diabetes history, hypoglycemic event history, etc. In some embodiments, user data may be stored elsewhere (e.g., at the mobile device 24 (FIG. 1), the vehicle processing unit 12 (FIG. 1), or remotely (e.g., in a storage device in the cloud(s) 18, 26 (FIG. 1)).

The software in memory 38 comprises a source program, executable program (object code), script, or any other entity comprising a set of instructions to be performed. When a source program, then the program may be translated via a compiler, assembler, interpreter, or the like, so as to operate properly in connection with the operating system. Furthermore, the software can be written as (a) an object oriented programming language, which has classes of data and methods, or (b) a procedure programming language, which has routines, subroutines, and/or functions, for example but not limited to, C, C+ +, Python, Java, among others. The software may be embodied in a computer program product, which may be a non-transitory computer readable medium or other medium.

The input interface(s) 52 comprises one or more interfaces (e.g., including a user interface) for entry of user input, such as a button or microphone or sensor(s) (e.g., to detect user input, including as a touch-type display screen). In some embodiments, the input interface 52 may serve as a communications port for downloaded information to the wearable device 22 (e.g., such as via a wired connection). The output interface(s) 54 comprises one or more interfaces for presenting feedback or data transfer (e.g., wired), including a user interface (e.g., display screen presenting a graphical or other type of user interface, virtual or augmented reality interface, etc.) or communications interface/port for the transfer (e.g., wired) of information stored in the memory 38. The output interface 54 may comprise other types of feedback devices, such as lighting devices (e.g., LEDs), audio devices (e.g., tone generator and speaker, buzzer), and/or tactile feedback devices (e.g., vibratory motor) and/or electrical feedback devices.

Referring now to FIG. 3, shown is an example mobile device 24 in which all or a portion of the functionality of a vehicle hypoglycemic event detection system may be implemented. In particular, FIG. 3 illustrates an example architecture (e.g., hardware and software) for the mobile device 24. It should be appreciated by one having ordinary skill in the art in the context of the present disclosure that the architecture of the mobile device 24 depicted in FIG. 3 is but one example, and that in some embodiments, additional, fewer, and/or different components may be used to achieve similar and/or additional functionality. In the depicted example, the mobile device 24 is embodied as a smartphone, though in some embodiments, other types of devices may be used, including a workstation, laptop, notebook, tablet, etc. The mobile device 24 may be used in some embodiments to provide the entire functionality of certain embodiments of a vehicle hypoglycemic event detection system, or in some embodiments, provide functionality of the vehicle hypoglycemic event detection system in conjunction with one or any combination of the wearable device 22 (FIG. 2), the vehicle processing unit 12 (FIG. 1), or one or more devices of the cloud(s) 18, 26 (FIG. 1), or other devices.

The mobile device 24 comprises at least two different processors, including a baseband processor (BBP) 56 and an application processor (APP) 58. As is known, the baseband processor 56 primarily handles baseband communication-related tasks and the application processor 58 generally handles inputs and outputs and all applications other than those directly related to baseband processing. The baseband processor 56 comprises a dedicated processor for deploying functionality associated with a protocol stack (PROT STK), such as but not limited to a GSM (Global System for Mobile communications) protocol stack, among other functions. The application processor 58 comprises a multi-core processor for running applications, including all or a portion of application software 40B. The baseband processor 56 and the application processor 58 have respective associated memory (MEM) 60, 62, including random access memory (RAM), Flash memory, etc., and peripherals, and a running clock. The memory 60, 62 are each also referred to herein also as a non-transitory computer readable medium. Note that, though depicted as residing in memory 62, all or a portion of the modules of the application software 40B may be stored in memory 60, distributed among memory 60, 62, or reside in other memory.

The baseband processor 56 may deploy functionality of the protocol stack to enable the mobile device 24 to access one or a plurality of wireless network technologies, including WCDMA (Wideband Code Division Multiple Access), CDMA (Code Division Multiple Access), EDGE (Enhanced Data Rates for GSM Evolution), GPRS (General Packet Radio Service), Zigbee (e.g., based on IEEE 802.15.4), Bluetooth, Wi-Fi (Wireless Fidelity, such as based on IEEE 802.11), and/or LTE (Long Term Evolution), among variations thereof and/or other telecommunication protocols, standards, and/or specifications. The baseband processor 56 manages radio communications and control functions, including signal modulation, radio frequency shifting, and encoding. The baseband processor 56 comprises, or may be coupled to, a radio (e.g., RF front end) 64 and/or a GSM (or other communications standard) modem, and analog and digital baseband circuitry (ABB, DBB, respectively in FIG. 3). The radio 64 comprises one or more antennas, a transceiver, and a power amplifier to enable the receiving and transmitting of signals of a plurality of different frequencies, enabling access to a cellular (and/or wireless) network. The analog baseband circuitry is coupled to the radio 64 and provides an interface between the analog and digital domains of the, for instance, GSM modem. The analog baseband circuitry comprises circuitry including an analog-to-digital converter (ADC) and digital-to-analog converter (DAC), as well as control and power management/distribution components and an audio codec to process analog and/or digital signals received indirectly via the application processor 58 or directly from a user interface (UI) 66 (e.g., microphone, touch-screen, though in some embodiments, may include an earpiece, ring tone, vibrator circuits, etc.). The ADC digitizes any analog signals for processing by the digital baseband circuitry. The digital baseband circuitry deploys the functionality of one or more levels of the GSM protocol stack (e.g., Layer 1, Layer 2, etc.), and comprises a microcontroller (e.g., microcontroller unit or MCU, also referred to herein as a processor) and a digital signal processor (DSP, also referred to herein as a processor) that communicate over a shared memory interface (the memory comprising data and control information and parameters that instruct the actions to be taken on the data processed by the application processor 58). The MCU may be embodied as a RISC (reduced instruction set computer) machine that runs a real-time operating system (RTIOS), with cores having a plurality of peripherals (e.g., circuitry packaged as integrated circuits) such as RTC (real-time clock), SPI (serial peripheral interface), I2C (inter-integrated circuit), UARTs (Universal Asynchronous Receiver/Transmitter), devices based on IrDA (Infrared Data Association), SD/MMC (Secure Digital/Multimedia Cards) card controller, keypad scan controller, and USB devices, GPRS crypto module, TDMA (Time Division Multiple Access), smart card reader interface (e.g., for the one or more SIM (Subscriber Identity Module) cards), timers, and among others. For receive-side functionality, the MCU instructs the DSP to receive, for instance, in-phase/quadrature (I/Q) samples from the analog baseband circuitry and perform detection, demodulation, and decoding with reporting back to the MCU. For transmit-side functionality, the MCU presents transmittable data and auxiliary information to the DSP, which encodes the data and provides to the analog baseband circuitry (e.g., converted to analog signals by the DAC).

The application processor 58 operates under control of an operating system (OS) that enables the implementation of a plurality of user applications, including the application software 40B. The application processor 58 may be embodied as a System on a Chip (SOC), and supports a plurality of multimedia related features including web browsing/cloud-based access functionality to access one or more computing devices, of the cloud(s) 18, 26 (FIG. 1), that are coupled to the Internet. For instance, the application processor 58 may execute communications functionality of the application software 40B (e.g., middleware, similar to some embodiments of the wearable device 22, which may include a browser with or operable in association with one or more application program interfaces (APIs)) to enable access to a cloud computing framework or other networks to provide remote data access/storage/processing, and through cooperation with an embedded operating system, access to calendars, location services, user data (e.g., private medical data), public data (population-based research or medical data), etc. The application processor 58 generally comprises a processor core (Advanced RISC Machine or ARM), and further comprises or may be coupled to multimedia modules (for decoding/encoding pictures, video, and/or audio), a graphics processing unit (GPU), communications interface (COMM) 68, and device interfaces. In one embodiment, the communications interfaces 68 may include wireless interfaces, including a Bluetooth (BT) (and/or Zigbee in some embodiments, among others) module that enable wireless communication with the wearable device 22, other mobile devices, and/or the vehicle processing unit 12 (FIG. 1). In some embodiments, the communications interface 68 may comprise a Wi-Fi module for interfacing with a local 802.11 network, according to corresponding communications software in the applications software 40B. The application processor 58 further comprises, or in the depicted embodiment, is coupled to, a global navigation satellite systems (GNSS) receiver 70 for enabling access to a satellite network to, for instance, provide position coordinates. In some embodiments, the GNSS receiver 70, in association with GNSS functionality in the application software 40B or otherwise stored in memory 62, collects time and location data, including location coordinates and altitude, velocity, etc. to ascertain driving behavior. Note that, though described as a GNSS receiver 70, other indoor/outdoor positioning systems may be used, including those based on triangulation of cellular network signals and/or Wi-Fi.

The device interfaces coupled to the application processor 58 may include the user interface 66, including a display screen. The display screen, in some embodiments similar to a display screen of the wearable device user interface, may be embodied in one of several available technologies, including LCD or Liquid Crystal Display (or variants thereof, such as Thin Film Transistor (TFT) LCD, In Plane Switching (IPS) LCD)), light-emitting diode (LED)-based technology, such as organic LED (OLED), Active-Matrix OLED (AMOLED), retina or haptic-based technology, or virtual/augmented reality technology. For instance, the user interface 66 may present visual feedback in the form of messaging (e.g., text messages) and/or symbols/graphics (e.g., warning or alert icons, flashing screen, etc.), and/or flashing lights (LEDs). In some embodiments, the user interface 66 may be configured, in addition to or in lieu of a display screen, a keypad, microphone, speaker, ear piece connector, I/O interfaces (e.g., USB (Universal Serial Bus)), SD/MMC card, among other peripherals. For instance, the speaker may be used to audibly provide feedback (e.g., voice, beeping sounds, buzzers, music or tones, etc.), and/or the user interface 66 may comprise a vibratory motor that provides a vibrating feedback to the user. One or any combination of visual, audible, or tactile feedback (alerts) may be used. In some embodiments, variations in the intensity of the feedback may be used to provide increasing awareness of the probability of a hypoglycemic event. For instance, color levels on the screen, buzzer or beeping sounds emitted from a speaker, tactile vibration frequency or strength, among other distinctions, may vary depending on the proximity to the actual event. As an example, multiple probability thresholds may be used with progressively increasing levels of feedback intensity. A similar approach may be used for alerts provided by the wearable device 22 and/or other devices disclosed herein.

Also coupled to the application processor 58 is an image capture device (IMAGE CAPTURE) 72. The image capture device 72 comprises an optical sensor (e.g., a charged coupled device (CCD) or a complementary metal-oxide semiconductor (CMOS) optical sensor). In one embodiment, the image capture device 72 may be configured as thermal imaging camera and/or a Vital Signs Camera, as described above. In general, the image capture device 72 may be used to detect various physiological parameters of a user and behavior (e.g., driving behavior), including blood pressure (e.g., based on remote photoplethysmography (PPG)), blood circulation, heart rate, respiration rate and/or breathing patterns, skin surface changes (e.g., pale skin, clammy skin), skin temperature, etc. Also included is a power management device 74 that controls and manages operations of a battery 76. The components described above and/or depicted in FIG. 3 share data over one or more busses, and in the depicted example, via data bus 78. It should be appreciated by one having ordinary skill in the art, in the context of the present disclosure, that variations to the above may be deployed in some embodiments to achieve similar functionality.

In the depicted embodiment, the application processor 58 runs the application software 40B, which in one embodiment, comprises a sensor measurement module 42B, prediction engine 44B comprising a learning (LEARN) module 46B, and a communications/feedback (CFB) module 48B. The application software 40B and associated modules 42B-48B are similar in functionality, for implementing an embodiment of a vehicle hypoglycemic event detection system, as described for the application software 40A of the wearable device 22 (FIG. 2), and hence description from the wearable device 22 above is applicable or largely applicable to the mobile device 24. The sensor measurement module 42B receives physiological parameters and/or other data (e.g., driving-style data) from sensors of the mobile device 24, including from the image capture device 72 and GNSS receiver 70. For instance, as described above, the image capture device 72 may monitor physiological signs of the driver including blood circulation, heart rate, and respiration rate, the corresponding data (hypoglycemic indicators) received at the sensor measurement module 42B. The image capture device 72 may also capture certain visual hypoglycemic indicators, including skin characteristics, eye motion, and/or tremors/shakiness of the driver, and convey the corresponding data to the sensor measurement module 42B. The image capture device 72, when positioned (e.g., on a console bracket) to monitor the face and torso of the driver, monitors a style of driving (also referred to as vehicle operation related parameters) of the driver and hence provides a basis for estimation (by the prediction engine 44B) of motor and cognitive performance (e.g., speed, acceleration, braking, cornering, odd movements, and distance to other cars). The GNSS receiver 70 may also be used to monitor driver behavior (e.g., through determinations of speed, direction, acceleration, etc.) alone, or in combination with the image capture device 72. The prediction engine 44B and learning module 46B receive one or more input parameters comprising the sensor data from the sensor measurement module 42B, and possibly other and/or additional data (e.g., from the cloud(s) 18, 26, wearable device 22, camera 30, external sensors 14, and/or internal sensors 16), and predicts when a hypoglycemic event is about to occur (e.g., has reached a threshold probability of occurring). As with the wearable device 22, multiple probability thresholds may be used in some embodiments, providing for multiple alerts in progressive fashion. The prediction engine 44B and learning module 46B determine baselines and thresholds in a similar manner as described above for like software modules (e.g., 44A, 46A) of the wearable device 22, and hence description of the same is omitted here for brevity. The communications/feedback module 48B provides for visual (e.g., text and/or color or graphics on a display screen of the mobile device 24, light patterns, etc.), audible (voice, beeper, buzzer, etc.), and/or tactile (e.g., vibration) feedback to the user via the UI 66 based on the triggering output of the prediction engine 44B. The communications/feedback module 48B may also, or alternatively, send a signal(s) to one or more other devices (e.g., the vehicle processing unit 12, the wearable device 22, headsets, etc.) to trigger similar feedback. In some embodiments, the communications/feedback module 48B may signal the vehicle processing unit 12 to activate one or more vehicle operating devices (e.g., braking, steering, semi-autonomous or autonomous systems) to change operations of the vehicle 10 (FIG. 1).

Referring now to FIG. 4, shown is an embodiment of an example vehicle processing unit 12 in which in which all or a portion of the functionality of a vehicle hypoglycemic event detection system may be implemented. Functionality of the vehicle processing unit 12 may be implemented alone, or in some embodiments, in combination with one or more additional devices. In one embodiment, the vehicle processing unit 12 may be embodied as a computer, though in some embodiments, may be embodied as an application server (e.g., if functionality of the vehicle occupant interaction system is implemented primarily remotely, such as in the cloud(s) 18 or 26, FIG. 1). One having ordinary skill in the art should appreciate in the context of the present disclosure that the example vehicle processing unit 12 is merely illustrative of one embodiment, and that some embodiments may comprise fewer or additional components. The vehicle processing unit 12 is depicted in this example as a computer system. It should be appreciated that certain well-known components of computer systems are omitted here to avoid obfuscating relevant features of the vehicle processing unit 12. In one embodiment, the vehicle processing unit 12 comprises hardware and software components, including one or more processors (one shown), such as processor (PROCESS) 80, input/output (I/O) interface(s) 82 (I/O), and memory 84 (MEM), all coupled to one or more data busses, such as data bus 86 (DBUS). The memory 84 (also referred to herein as a non-transitory computer readable medium) may include any one or a combination of volatile memory elements (e.g., random-access memory RAM, such as DRAM, and SRAM, etc.) and nonvolatile memory elements (e.g., ROM, Flash, solid state, EPROM, EEPROM, hard drive, tape, CDROM, etc.). The memory 84 may store a native operating system (OS), one or more native applications, emulation systems, or emulated applications for any of a variety of operating systems and/or emulated hardware platforms, emulated operating systems, etc. In some embodiments, one or more separate storage devices (STOR DEV) may be coupled to the data bus 86 and/or the vehicle processing unit 12 may be coupled to network storage (e.g., which may be part of the cloud(s) 18, 26, FIG. 1) via a network (NW) and communications functionality as described further below.

In the depicted embodiment, the vehicle processing unit 12 is coupled via the I/O interfaces 82 to a communications interface (COM) 88, a user interface (UI) 90, and one or more sensors 92. In some embodiments, the communications interface 88, user interface 90, and one or more sensors 92 may be coupled directly to the data bus 86. The communications interface 88 comprises hardware and software for wireless functionality (e.g., Bluetooth, near field communications, Wi-Fi, etc.), enabling wireless communications with devices located internal to the vehicle 10 (FIG. 1), including the wearable device 22 (FIG. 2), mobile device 24 (FIG. 3), among other devices (e.g., camera 30), and optionally wireless communications with sensors 92 of the vehicle 10 that are located on the exterior of the vehicle 10. In one embodiment, the communications interface 88 further comprises cellular modem functionality to enable cellular communications to access computing functionality of the cloud(s) 18, 26, such as to access public or proprietary data structures (e.g., databases). For instance, a user profile may be located in one or more devices of the cloud(s) 18, 26, and includes user data (e.g., history of hypoglycemic indicators, medical history, including diabetes and/or hypoglycemic event history, etc.) of the driver and/or public statistics or information (e.g., population statistics and/or research on hypoglycemia indicators, environmental data, including weather data). In some embodiments, the weather data may be acquired via sensors 92 located within (or located on the exterior of the vehicle 10), or via stand-alone devices found within the vehicle 10, including through the use of a netamo device. In some embodiments, one or more of the information may be stored locally for a transitory period (e.g., in storage device and/or memory 84), including baseline data, thresholds, etc.

The I/O interfaces 82 may comprise any number of interfaces for the input and output of signals (e.g., analog or digital data) for conveyance of information (e.g., data) over various networks and according to various protocols and/or standards.

The user interface 90 comprises one or any combination of a display screen with or without a graphical user interface (GUI), heads-up display, keypad, vehicle buttons/switches/knobs or other mechanisms to enable the entry of user commands for the vehicle controls, microphone, mouse, etc., and/or feedback to the driver and/or passenger. For instance, the user interface 90 may include dedicated lighting (e.g., internal status lights, such as a warning light or caution light or pattern) or other mechanisms to provide visual feedback/alerts, including a console display having emoji icons or other symbolic graphics or even text warnings. In some embodiments, the user interface 90 comprises one or more vibratory motors (e.g., in the driver and/or passenger seat, stick-shift, steering wheel, arm rest, etc.) to provide tactile feedback to the driver and/or passenger within the vehicle 10 (FIG. 1), such as to warn the passenger that the driver is about to experience a hypoglycemic event. In some embodiments, the user interface 90 comprises speakers and/or microphones, such as to provide beeping, buzzard, or other sounds (e.g., tones, or verbal speaking) that warn the driver of an impending hypoglycemic event. The intensity of the various feedback may also be altered, such as increasing frequency or volume of sounds as the condition worsens in some embodiments (e.g., by using more than one threshold probability). Note that one or any combination of the various feedback techniques and/or devices described above may be used at any one time. In some embodiments, feedback may be performed via other devices (e.g., the wearable device 22, mobile device 24, feedback devices within the vehicle 10, etc.), for instance when triggered by wireless or wired communications from the vehicle processing unit 12.

The sensors 92 comprise internal and external sensors (e.g., internal sensors 16 and external sensor 14, FIG. 1), including camera sensors (e.g., camera 30, FIG. 1) and/or position locating sensors (e.g., GNSS receiver). The sensors 92 include the vehicle sensors that are associated with vehicle motion, including one or any combination of inertial motion sensors (e.g., gyroscopes, magnetometers), the GNSS receiver, load sensors, position sensors, velocity sensors, and/or acceleration sensors. In other words, the sensors 92 measure the vehicle movement information associated with the driver's style of driving, including the abruptness of starts and stops, fast accelerations, speed, sharp turns, and/or odd movements. In some embodiments, the sensors 92 may be located in devices that are in wired or wireless communication with the vehicle processing unit 12. For instance, and as indicated above, the sensors 92 may include touch and/or grip sensors integrated in the steering wheel (e.g., to detect changes in physiological signals of the driver, including heart rate, acceleration, perspiration, clammy skin, etc.). In some embodiments, the sensors 92 may include touch/force handles integrated in the steering wheel to detect tremors/shakiness of the driver. In some embodiments, the sensors 92 may include grip sensors integrated into the shifting gears to detect changes in physiological signals of the driver, including tremors/shakiness. In some embodiments, the sensors 92 may include motion sensors (e.g., accelerometers) and/or force sensors to detect motion, including shaking/tremors and/or perspiration. In some embodiments, the sensors 92 may include a glucose reading sensor(s), which may be integrated into the internal structure of the vehicle 10, including in the steering wheel, enabling a better estimation of the risk of a hypoglycemic event (e.g., especially in a diabetic person). In some embodiments, other input may include the time of insulin intake by the driver (e.g., as recorded in the cloud(s) and accessed by the vehicle processing unit, or accessed from via the mobile device 24 and/or wearable device 22, enabling an estimation of when a hypoglycemic event is about to occur.

In the embodiment depicted in FIG. 4, the memory 84 comprises an operating system (OS) and application software (ASW) 40C. Note that in some embodiments, the application software 40C may be implemented without the operating system. In one embodiment, the application software 40C comprises a sensor measurement module 42C, a prediction engine 44C, including a learning (LEARN) module 46C, and a communications/feedback (CFB) module 48C. The application software 40C and associated modules 42C-48C are similar, in functionality for implementing an embodiment of a vehicle hypoglycemic event detection system, as described for the application software 40A of the wearable device 22 (FIG. 2), and hence description from the wearable device 22 above is applicable or largely applicable to the vehicle processing unit 12. The sensor measurement module 42C receives physiological parameters and/or other data (e.g., driving-style data) from the sensors 92. The prediction engine 44C and learning module 46C receive input parameters comprising the sensor data from the sensor measurement module 42C, and possibly other and/or additional data (e.g., from the cloud(s) 18, 26, wearable device 22, mobile device 24) and predicts when a hypoglycemic event is about to occur (e.g., has reached a threshold probability of occurring). The prediction engine 44C and learning module 46C determine baselines and thresholds in a similar manner as described above for like software modules (e.g., 44A, 46A) of the wearable device 22, and hence description of the same is omitted here for brevity. The communications/feedback module 48C provides for visual (e.g., text and/or color or graphics on a dashboard or other display screen of the vehicle 10), audible (voice, beeper, buzzer, etc.), and/or tactile (e.g., vibration) feedback to the user via the UI 90 based on the triggering output of the prediction engine 44C. The communications/feedback module 48C may also, or alternatively, send a signal(s) to one or more other devices (e.g., the mobile device 24, the wearable device 22, headsets, Google Glass, etc.) to trigger similar feedback. In some embodiments, the communications/feedback module 48C may activate one or more vehicle operating devices (e.g., braking, steering, semi-autonomous or autonomous systems) to change operations of the vehicle 10. The communications/feedback module 48C generally enables (via the communications interface 88) communications among devices connected to one or more networks (NW) (e.g., personal area network, local wireless area network, wide area network, cellular network, etc.), including enabling web-browsing and/or access to cloud services through the use of one or more APIs.

Execution of the application software 40C may be implemented by the processor 80 under the management and/or control of the operating system (or in some embodiments, without the use of the OS). The processor 80 (or processors) may be embodied as a custom-made or commercially available processor, a central processing unit (CPU) or an auxiliary processor among several processors, a semiconductor based microprocessor (in the form of a microchip), a macroprocessor, one or more application specific integrated circuits (ASICs), a plurality of suitably configured digital logic gates, and/or other well-known electrical configurations comprising discrete elements both individually and in various combinations to coordinate the overall operation of the vehicle processing unit 12.

When certain embodiments of the vehicle processing unit 12 are implemented at least in part with software (including firmware), as depicted in FIG. 4, it should be noted that the software can be stored on a variety of non-transitory computer-readable medium for use by, or in connection with, a variety of computer-related systems or methods. In the context of this document, a computer-readable medium may comprise an electronic, magnetic, optical, or other physical device or apparatus that may contain or store a computer program (e.g., executable code or instructions) for use by or in connection with a computer-related system or method. The software may be embedded in a variety of computer-readable mediums for use by, or in connection with, an instruction execution system, apparatus, or device, such as a computer-based system, processor-containing system, or other system that can fetch the instructions from the instruction execution system, apparatus, or device and execute the instructions.

When certain embodiments of the vehicle processing unit 12 are implemented at least in part with hardware, such functionality may be implemented with any or a combination of the following technologies, which are all well-known in the art: a discrete logic circuit(s) having logic gates for implementing logic functions upon data signals, an application specific integrated circuit (ASIC) having appropriate combinational logic gates, a programmable gate array(s) (PGA), a field programmable gate array (FPGA), relays, contactors, etc.

Note that the functionality of the application software 40 (e.g., 40A, 40B, 40C) may be implemented in a single device located internal or external to the vehicle 10, or in some embodiments, be distributed among two or more of such devices. Input parameters to the prediction engine 44 (e.g., 44A, 44B, 44C) may be sourced from sensors from one or a plurality of devices (e.g., embedded in the device hosting the prediction engine 44, received as a fusion of sensors from a plurality of devices, and/or other input, including data from data structures external and internal to the vehicle 10). It is further noted that a device on the cloud(s) 18, 26 may have a similar architecture to the vehicle processing unit 12 in some embodiments.

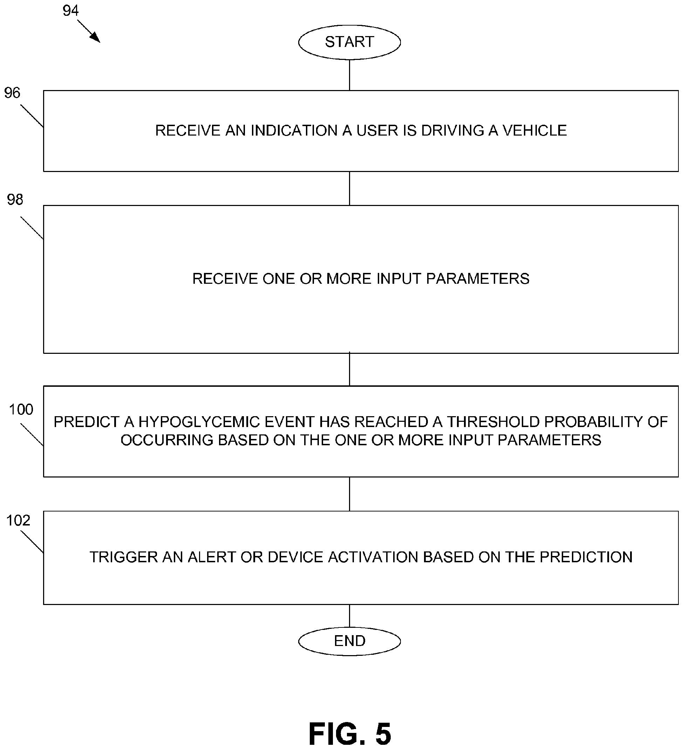

In view of the above-description, it should be appreciated by one having ordinary skill in the art, in the context of the present disclosure, that one embodiment of a method, denoted as method 94 and depicted in FIG. 5, comprises (beginning from start and ending at end) receiving an indication a user is driving a vehicle (96); receiving one or more input parameters (98); predicting a hypoglycemic event has reached a threshold probability of occurring based on the one or more input parameters (100); and triggering an alert or device activation based on the prediction (102). The method 94 may be implemented by the wearable device 22, mobile device 24, vehicle processing unit 12, and/or one or more devices of the cloud(s) 18, 26.

Any process descriptions or blocks in flow diagrams should be understood as representing modules, segments, or portions of code which include one or more executable instructions for implementing specific logical functions or steps in the process, and alternate implementations are included within the scope of the embodiments in which functions may be executed out of order from that shown or discussed, including substantially concurrently or in reverse order, depending on the functionality involved, as would be understood by those reasonably skilled in the art of the present disclosure.

The invention is defined by the appended claims.

## Claims

1. An apparatus (22), comprising:
a memory (38) with instructions (40A); and
one or more processors (36), wherein the one or more processors are configured to execute the instructions to:
receive an indication a user is present in an internal vehicle environment;
receive input parameters from sensors, wherein the input parameters comprise a combination of (i) one or more physiological input parameters corresponding to the user and (ii) one or more internal vehicle environmental parameters; **characterized in that** the one or more processors are configured to
evaluate the one or more internal vehicle environmental parameters to validate whether one or any combination of the physiological input parameters is based on hypoglycemia or on the internal environmental conditions in the vehicle;
determine a potential onset of a hypoglycemic event based on a result of the validation; and
trigger an alert or device activation based on the determination.

2. The apparatus of claim 1, wherein the one or more input parameters further comprise one or more vehicle operation related input parameters.

3. The apparatus of claim 1 or 2, wherein the one or more physiological input parameters comprises an indication of one or any combination of blood circulation changes, vasoconstriction changes, increase in shakiness of the user, heart rate increase, respiration increase, temperature decrease, eye motion changes, skin conductance increase, a glucose reading, or insulin intake history.

4. The apparatus of claim 3, wherein the one or more processors are further configured to execute the instructions to validate whether one or any combination of the temperature decrease or skin conductance increase is based on hypoglycemia or internal environmental conditions in the vehicle by evaluating one or more internal vehicle environmental parameters.

5. The apparatus of claim 2, wherein the one or more vehicle operation related input parameters comprises one or any combination of motion information about the vehicle, motion information relative to one or more other vehicles, or driving behavior of the user.

6. The apparatus of any of the preceding claims, further comprising one or more sensors (32).

7. The apparatus of claim 6, wherein the one or more sensors comprises one or any combination of an accelerometer, a camera, or a position locating device.

8. The apparatus of any of the preceding claims, wherein the one or more processors are further configured to execute the instructions to receive the one or more input parameters while the user is driving and before the user is driving.

9. The apparatus of any of the preceding claims, wherein the one or more processors are further configured to execute the instructions to predict based on a comparison between the one or more input parameters and one or more respective thresholds.

10. The apparatus of claim 9, wherein the one or more respective thresholds are adaptive based on one or any combination of historical user data or a health status of the user.

11. The apparatus of any of the preceding claims, wherein the one or more processors are further configured to execute the instructions to trigger the alert by communicating a signal that triggers one or any combination of a visual, audible, or haptic warning to the user.

12. The apparatus of any of the preceding claims, wherein the one or more processors are further configured to execute the instructions to trigger the device activation by communicating a signal that adjusts a device within the vehicle that effects a change in operation of the vehicle.

13. The apparatus of any of the preceding claims, wherein the memory and processor are contained in a wearable device, a mobile device, a device located external to the vehicle, or a device located internal to the vehicle.

14. A method (94), comprising:
receiving an indication a user is present in an internal vehicle environment (96);
receiving one or more input parameters (98), wherein the one or more input parameters comprise a combination of (i) one or more physiological input parameters corresponding to the user and (ii) one or more internal vehicle environmental parameters, **characterized in that** the method further comprises:
evaluating the one or more internal vehicle environmental parameters to validate whether one or any combination of the physiological input parameters is based on hypoglycemia or on the internal environmental conditions in the vehicle;
determining a potential onset of a hypoglycemic event based on a result of the validation (100); and
triggering an alert or device activation based on the determination (102).

## Patentansprüche

1. Vorrichtung (22), umfassend:
einen Speicher (38) mit Anweisungen (40A); und
einen oder mehrere Prozessoren (36),
wobei der eine oder die mehreren Prozessoren konfiguriert sind, um die Anweisungen zu Folgendem auszuführen:
Empfangen einer Angabe, dass ein Benutzer in einer inneren Fahrzeugumgebung anwesend ist;
Empfangen von Eingangsparametern von Sensoren, wobei die Eingangsparameter eine Kombination aus (i) einem oder mehreren physiologischen Eingangsparametern, die dem Benutzer entsprechen, und (ii) einem oder mehreren internen Fahrzeugumgebungsparametern umfassen;
**dadurch gekennzeichnet, dass** der eine oder die mehreren Prozessoren konfiguriert sind, um den einen oder die mehreren internen Fahrzeugumgebungsparameter auszuwerten, um zu validieren, ob einer oder eine beliebige Kombination der physiologischen Eingangsparameter auf einer Hypoglykämie oder auf den internen Umgebungsbedingungen in dem Fahrzeug basiert;
Bestimmen eines möglichen Einsetzens eines hypoglykämischen Ereignisses basierend auf einem Resultat der Validierung; und
Auslösen eines Alarms oder einer Vorrichtungsaktivierung basierend auf der Bestimmung.

2. Vorrichtung nach Anspruch 1, wobei der eine oder die mehreren Eingangsparameter ferner einen oder mehrere fahrzeugbetriebsbezogene Eingangsparameter umfassen.

3. Vorrichtung nach Anspruch 1 oder 2, wobei der eine oder die mehreren physiologischen Eingangsparameter einen Hinweis auf eine oder eine beliebige Kombination von Blutkreislaufänderungen, Gefäßverengungsänderungen, Erhöhung einer Zittrigkeit des Benutzers, Herzfrequenzerhöhung, Atmungserhöhung, Temperaturverringerung, Augenbewegungsänderungen, Hautleitwerterhöhung, einen Glukosewert oder einen Insulineinnahmeverlauf umfassen.

4. Vorrichtung nach Anspruch 3, wobei der eine oder die mehreren Prozessoren ferner konfiguriert sind, um die Anweisungen auszuführen, um durch Auswerten einer oder mehrerer interner Umgebungsbedingungen in dem Fahrzeug zu validieren, ob eine oder eine beliebige Kombination aus Temperaturverringerung oder Hautleitwerterhöhung auf Hypoglykämie oder internen Umgebungsbedingungen in dem Fahrzeug basiert.

5. Vorrichtung nach Anspruch 2, wobei der eine oder die mehreren fahrzeugbetriebsbezogenen Eingangsparameter eine oder eine beliebige Kombination von Bewegungsinformationen über das Fahrzeug, Bewegungsinformationen in Bezug auf ein oder mehrere andere Fahrzeuge oder das Fahrverhalten des Benutzers umfassen.

6. Vorrichtung nach einem der vorherigen Ansprüche, ferner umfassend einen oder mehrere Sensoren (32).

7. Vorrichtung nach Anspruch 6, wobei der eine oder die mehreren Sensoren einen oder eine beliebige Kombination aus einem Beschleunigungsmesser, einer Kamera oder einer Positionsbestimmungsvorrichtung umfassen.

8. Vorrichtung nach einem der vorherigen Ansprüche, wobei der eine oder die mehreren Prozessoren ferner konfiguriert sind, um die Anweisungen auszuführen, um den einen oder die mehreren Eingangsparameter zu empfangen, während der Benutzer fährt und bevor der Benutzer fährt.

9. Vorrichtung nach einem der vorherigen Ansprüche, wobei der eine oder die mehreren Prozessoren ferner konfiguriert sind, um die Anweisungen auszuführen, um basierend auf einem Vergleich zwischen dem einen oder den mehreren Eingangsparametern und einem oder mehreren entsprechenden Schwellenwerten eine Vorhersage zu treffen.

10. Vorrichtung nach Anspruch 9, wobei der eine oder die mehreren jeweiligen Schwellenwerte basierend auf einer oder einer beliebigen Kombination von historischen Benutzerdaten oder einem Gesundheitszustand des Benutzers adaptiv sind.

11. Vorrichtung nach einem der vorherigen Ansprüche, wobei der eine oder die mehreren Prozessoren ferner konfiguriert sind, um die Anweisungen auszuführen, um den Alarm auszulösen, indem sie ein Signal übermitteln, das eine oder eine beliebige Kombination aus einer visuellen, akustischen oder haptischen Warnung an den Benutzer auslöst.

12. Vorrichtung nach einem der vorherigen Ansprüche, wobei der eine oder die mehreren Prozessoren ferner konfiguriert sind, um die Anweisungen ausführen, um die Vorrichtungsaktivierung durch Übermitteln eines Signals auszulösen, das eine Vorrichtung innerhalb des Fahrzeugs einstellt, die eine Änderung im Betrieb des Fahrzeugs bewirkt.

13. Vorrichtung nach einem der vorherigen Ansprüche, wobei der Speicher und der Prozessor in einer tragbaren Vorrichtung, einer mobilen Vorrichtung, einer außerhalb des Fahrzeugs befindlichen Vorrichtung oder einer innerhalb des Fahrzeugs befindlichen Vorrichtung enthalten sind.

14. Verfahren (94), umfassend:
Empfangen einer Angabe, dass ein Benutzer in einer inneren Fahrzeugumgebung anwesend ist (96);
Empfangen eines oder mehrerer Eingangsparameter (98), wobei der eine oder die mehreren Eingangsparameter eine Kombination aus (i) einem oder mehreren physiologischen Eingangsparametern, die dem Benutzer entsprechen, und (ii) einem oder mehreren internen Fahrzeugumgebungsparametern umfassen,
**dadurch gekennzeichnet, dass** das Verfahren ferner Folgendes umfasst:
Auswerten des einen oder der mehreren internen Fahrzeugumgebungsparameter, um zu validieren, ob einer oder eine beliebige Kombination der physiologischen Eingangsparameter auf einer Hypoglykämie oder auf den internen Umgebungsbedingungen in dem Fahrzeug basiert;
Bestimmen eines möglichen Einsetzens eines hypoglykämischen Ereignisses basierend auf einem Resultat der Validierung (100); und
Auslösen eines Alarms oder einer Vorrichtungsaktivierung basierend auf der Bestimmung (102) .

## Revendications

1. Appareil (22), comprenant:
une mémoire (38) avec des instructions (40A); et
un ou plusieurs processeurs (36),
dans lequel l'un ou les plusieurs processeurs sont configurés pour exécuter les instructions pour:
recevoir une indication qu'un utilisateur est présent dans un environnement interne de véhicule;
recevoir des paramètres d'entrée à partir de capteurs, dans lequel les paramètres d'entrée comprennent une combinaison de (i) un ou plusieurs paramètres d'entrée physiologiques correspondant à l'utilisateur et (ii) un ou plusieurs paramètres environnementaux internes de véhicule;
**caractérisé en ce que** l'un ou les plusieurs processeurs sont configurés pour
évaluer l'un ou les plusieurs paramètres environnementaux internes de véhicule pour valider si un ou toute combinaison des paramètres d'entrée physiologiques est basé sur de l'hypoglycémie ou sur les conditions environnementales internes dans le véhicule;
déterminer un début potentiel d'un événement hypoglycémique sur la base d'un résultat de la validation; et
déclencher une alerte ou une activation de dispositif sur la base de la détermination.

2. Appareil selon la revendication 1, dans lequel l'un ou les plusieurs paramètres d'entrée comprennent en outre un ou plusieurs paramètres d'entrée liés au fonctionnement du véhicule.

3. Appareil selon la revendication 1 ou 2, dans lequel l'un ou les plusieurs paramètres d'entrée physiologiques comprennent une indication d'une ou de toute combinaison de changements de circulation sanguine, changements de vasoconstriction, augmentation des tremblements de l'utilisateur, augmentation de la fréquence cardiaque, augmentation de la respiration, diminution de la température, changements de mouvement des yeux, augmentation de la conductance cutanée, lecture du glucose ou historique de consommation d'insuline.

4. Appareil selon la revendication 3, dans lequel l'un ou les plusieurs processeurs sont en outre configurés pour exécuter les instructions pour valider si une ou toute combinaison de la diminution de la température ou de l'augmentation de la conductance cutanée est basée sur de l'hypoglycémie ou les conditions environnementales internes dans le véhicule en évaluant un ou plusieurs paramètres environnementaux internes de véhicule.

5. Appareil selon la revendication 2, dans lequel l'un ou les plusieurs paramètres d'entrée liés au fonctionnement du véhicule comprennent une ou toute combinaison d'informations de mouvement concernant le véhicule, informations de mouvement relatives à un ou plusieurs autres véhicules, ou comportement de conduite de l'utilisateur.

6. Appareil selon l'une quelconque des revendications précédentes, comprenant en outre un ou plusieurs capteurs (32).

7. Appareil selon la revendication 6, dans lequel l'un ou les plusieurs capteurs comprennent une ou toute combinaison d'un accéléromètre, d'une caméra ou d'un dispositif de localisation de position.

8. Appareil selon l'une quelconque des revendications précédentes, dans lequel l'un ou les plusieurs processeurs sont en outre configurés pour exécuter les instructions pour recevoir l'un ou les plusieurs paramètres d'entrée pendant que l'utilisateur conduit et avant que l'utilisateur ne conduise.

9. Appareil selon l'une quelconque des revendications précédentes, dans lequel l'un ou les plusieurs processeurs sont en outre configurés pour exécuter les instructions à prédire sur la base d'une comparaison entre l'un ou les plusieurs paramètres d'entrée et un ou plusieurs seuils respectifs.

10. Appareil selon la revendication 9, dans lequel l'un ou les plusieurs seuils respectifs sont adaptatifs sur la base d'une ou de toute combinaison de données d'utilisateur historiques ou d'un état de santé de l'utilisateur.

11. Appareil selon l'une quelconque des revendications précédentes, dans lequel l'un ou les plusieurs processeurs sont en outre configurés pour exécuter les instructions pour déclencher l'alerte en communiquant un signal qui déclenche une ou toute combinaison d'un avertissement visuel, audible ou haptique à l'utilisateur.

12. Appareil selon l'une quelconque des revendications précédentes, dans lequel l'un ou les plusieurs processeurs sont en outre configurés pour exécuter les instructions pour déclencher l'activation de dispositif en communiquant un signal qui ajuste un dispositif au sein du véhicule qui effectue un changement de fonctionnement du véhicule.

13. Appareil selon l'une quelconque des revendications précédentes, dans lequel la mémoire et le processeur sont contenus dans un dispositif portable, un dispositif mobile, un dispositif situé à l'extérieur du véhicule ou un dispositif situé à l'intérieur du véhicule.

14. Procédé (94), consistant à:
recevoir une indication qu'un utilisateur est présent dans un environnement interne de véhicule (96);
recevoir un ou plusieurs paramètres d'entrée (98),
dans lequel l'un ou les plusieurs paramètres d'entrée comprennent une combinaison de (i) un ou plusieurs paramètres d'entrée physiologiques correspondant à l'utilisateur et (ii) un ou plusieurs paramètres environnementaux internes de véhicule,
**caractérisé en ce que** le procédé comprend en outre:
évaluer l'un ou les plusieurs paramètres environnementaux internes de véhicule pour valider si un ou toute combinaison des paramètres d'entrée physiologiques est basé sur de l'hypoglycémie ou sur les conditions environnementales internes dans le véhicule;
déterminer un début potentiel d'un événement hypoglycémique sur la base d'un résultat de la validation (100); et
déclencher une alerte ou une activation de dispositif sur la base de la détermination (102).
